# EUROPÄISCHE PATENTANMELDUNG

(11) **EP 4 552 670 A1**
(43) Veröffentlichungstag der Anmeldung: **14.05.2025**
(21) Anmeldenummer: 23208191.9
(22) Anmeldetag: 07.11.2023
(51) Int. Cl.: A61M 5/168

(54) **MEDIZINISCHE VORRICHTUNG MIT AKUSTISCHER UND/ODER HAPTISCHER UND/ODER OPTISCHER RÜCKMELDUNG**

(71) Anmelder: B. Braun Melsungen AG, 34212 Melsungen (DE)
(72) Erfinder: AKKUS, Hidayet, 34119 Kassel (DE); KAUBA, Michael, 34277 Fuldabrück (DE); DIEHL, Johannes, 34212 Melsungen (DE); VOGEL, Markus, 37191 Katlenburg-Lindau (DE)
(74) Vertreter: Winter, Brandl - Partnerschaft mbB

(57) **Zusammenfassung**

Eine offenbarungsgemäße medizinische Vorrichtung (1) umfasst eine Eingabeeinheit (20), die ausgebildet ist, eine durch einen Nutzer (18) getätigte Eingabebetätigung (16) zum Bedienen der medizinischen Vorrichtung (1) zu empfangen, und eine Rückmeldeeinheit (32), die ausgebildet ist, dem Nutzer (18) in Erwiderung auf die Eingabebetätigung (16) eine haptische Rückmeldung (28) und/oder eine akustische Rückmeldung (30) und/oder eine optische Rückmeldung (31) auszugeben.

## Beschreibung

Die vorliegende Offenbarung betrifft eine medizinische Vorrichtung mit einer Eingabeeinheit zum Empfangen einer durch einen Nutzer getätigten Eingabebetätigung und einer Rückmeldeeinheit zum Ausgeben einer Rückmeldung in Erwiderung auf die Eingabebetätigung. Die medizinische Vorrichtung kann insbesondere eine Infusionspumpe sein.

### Stand der Technik

Derzeit werden in einer medizinischen Vorrichtung fast ausschließlich ein resistiver Touchscreen und/oder mechanische/haptische Tasten verbaut, um eine Eingabebetätigung durch einen Nutzer zum Bedienen der medizinischen Vorrichtung zu empfangen. Die über den resistiven Touchscreen oder eine mechanische Taste getätigte Eingabebetätigung wird mittels eines redundanten oder sogar diversitären Erfassungssystems erfasst, um eine hohe Sicherheit beim Empfangen der Eingabebetätigung sicherzustellen. Dies führt zu einem gesteigerten Bedarf an Komponenten bzw. Software und im Fall des diversitären Erfassungssystems sogar zu einem erhöhten Entwicklungsaufwand. Zudem stellt ein resistiver Touchscreen nur eine begrenzte Auflösung zum Definieren von Bereichen zum Empfangen unterschiedlicher Eingabebetätigungen bereit, so dass oft zwischen unterschiedlichen Anzeigen auf dem resistiven Touchscreen gewechselt werden muss.

### Kurzbeschreibung der Offenbarung

Es ist daher Aufgabe der vorliegenden Offenbarung, diese Nachteile zu beseitigen oder zumindest zu mindern. Es ist insbesondere Aufgabe der vorliegenden Offenbarung, eine medizinische Vorrichtung zu schaffen, die eine hohe Sicherheit beim Empfangen einer durch einen Nutzer getätigten Eingabebetätigung zum Bedienen der medizinischen Vorrichtung gewährleistet.

Die Aufgabe wird durch die medizinische Vorrichtung mit den Merkmalen gemäß Anspruch 1 gelöst. Vorteilhafte Ausführungsformen sind Gegenstand der abhängigen Ansprüche und/oder werden nachfolgend erläutert.

Eine offenbarungsgemäße medizinische Vorrichtung weist eine Eingabeeinheit, die ausgebildet ist, eine durch einen Nutzer getätigte Eingabebetätigung zum Bedienen der medizinischen Vorrichtung zu empfangen, auf. Über die Eingabebetätigung kann eine mit der medizinischen Vorrichtung durchgeführte Behandlung gesteuert und/oder parametriert werden, d.h. die Behandlung kann gestartet oder gestoppt werden und es können Parameter in Bezug auf die Behandlung vor oder während der Behandlung eingestellt werden. Die medizinische Vorrichtung kann insbesondere eine Infusionspumpe sein. Alternativ kann die medizinische Vorrichtung eine extrakorporale Blutbehandlungsvorrichtung, bevorzugt eine Dialysemaschine, oder eine Steuervorrichtung für ein medizinisches oder chirurgisches Werkzeug, z.B. einen Bohrer, eine Fräse oder eine Säge, sein. Eine Vielzahl von weiteren, alternativen Ausgestaltungen der medizinischen Vorrichtung sind denkbar.

Die medizinische Vorrichtung weist außerdem eine Rückmeldeeinheit auf, die ausgebildet ist, dem Nutzer in Erwiderung auf die Eingabebetätigung eine Rückmeldung auszugeben bzw. zurückzugeben. Die Rückmeldung kann dabei eine haptische bzw. mechanische Rückmeldung oder eine akustische Rückmeldung oder eine Kombination aus haptischer und akustischer Rückmeldung sein. In anderen Worten kann die Rückmeldung eine haptische bzw. mechanische Rückmeldung und/oder eine akustische Rückmeldung sein. Folglich ist die Rückmeldeeinheit imstande, nur eine akustische Rückmeldung oder nur eine haptische/ mechanische Rückmeldung auszugeben. Die Rückmeldeeinheit kann aber auch eine Kombination aus einer akustischen und einer haptischen/ mechanischen Rückmeldung ausgeben. Es ist auch denkbar, dass die Rückmeldeeinheit zusätzlich oder alternativ zu der akustischen und/oder haptischen Rückmeldung eine optische Rückmeldung, beispielsweise in Form eines Lichtsignals z.B. von einer LED oder einer Anzeige auf einem Touchscreen, ausgibt. Auch eine Kombination aus einer optischen Rückmeldung mit einer akustischen Rückmeldung und/oder einer haptischen Rückmeldung ist somit denkbar, genauso wie eine rein optische Rückmeldung. Demnach erhält der Nutzer der medizinischen Vorrichtung unmittelbar nach Tätigen der Eingabebetätigung über die Eingabeeinheit eine Rückmeldung durch die Rückmeldeeinheit. Auf diese Weise wird dem Nutzer der medizinischen Vorrichtung der Empfang der Eingabebetätigung bestätigt, so dass eine Sicherheit beim Tätigen der Eingabebetätigung gewährleistet ist. Zusätzlich ist es nicht länger erforderlich, ein redundantes oder gar ein diversitäres Erfassungssystem zum Erfassen der Eingabebetätigung vorzusehen.

Bevorzugt kann die Eingabeeinheit ausgebildet sein, unterschiedliche Typen von Eingabebetätigungen zu empfangen. Demnach ist für jede Eingabebetätigung ein eigener Typ festlegbar. Der Typ kann dabei entsprechend von Sicherheitskriterien für die mit der medizinischen Vorrichtung durchgeführte Behandlung festgelegt sein. Die Rückmeldeeinheit kann dann so ausgebildet sein, dass sie jeweils unterschiedliche Typen von Rückmeldungen in Erwiderung auf die unterschiedlichen Typen von Eingabebetätigungen ausgibt. Folglich wird dem Nutzer nicht nur der Empfang der Eingabebetätigung, sondern auch der Typ der Eingabebetätigung durch die Rückmeldeeinheit zurückgegeben bzw. bestätigt, so dass eine Sicherheit beim Tätigen der Eingabebetätigung erhöht wird.

Mit Vorteil kann die Rückmeldeeinheit ausgebildet sein, dass eine Intensität oder Frequenz der haptischen Rückmeldung und/oder eine Lautstärke der akustischen Rückmeldung und/oder eine Helligkeit der optischen Rückmeldung festlegbar ist. Demnach kann der Nutzer der medizinischen Vorrichtung die Intensität/Frequenz bzw. die Lautstärke entsprechend seinen Präferenzen einstellen, um die Rückmeldung, die in Erwiderung auf die empfangene Eingabebetätigung ausgegeben wird, zuverlässig wahrzunehmen. Gleiches gilt für die Einstellung der Helligkeit der optischen Rückmeldung. Beispielsweise ist eine Festlegung einer erhöhten Intensität oder einer erhöhten Frequenz in einem Fall vorteilhaft, in dem der Nutzer Handschuhe während der Eingabebetätigung trägt. Somit kann eine Sicherheit beim Tätigen der Eingabebetätigung weiter erhöht werden.

Vorteilhafterweise können die Typen von Eingabebetätigungen in eine erste Gruppe gruppiert sein. Die Rückmeldeeinheit kann dann so ausgebildet sein, dass sie eine vorgegebene und nicht änderbare Rückmeldung in Erwiderung auf einen Typ von Eingabebetätigung der ersten Gruppe ausgibt. In die erste Gruppe können Typen von Eingabebetätigungen gruppiert werden, die bei einer großen Anzahl von unterschiedlichen medizinischen Vorrichtungen gleichsam vorhanden sind. Außerdem können in die erste Gruppe Typen von Eingabebetätigungen gruppiert werden, die eine hohe Relevanz für die Zuverlässigkeit bzw. Sicherheit bei einer mit der medizinischen Vorrichtung durchgeführten Behandlung haben. Beispiele hierfür sind eine Home-Taste, eine An/Aus-Taste, eine Stop-Taste oder eine Start-Taste. Beim Empfangen einer derartigen Eingabebetätigung wird immer dieselbe Rückmeldung zurückgegeben, so dass der Nutzer sofort die Durchführung dieser bestimmten Eingabebetätigung validieren kann. Zum Beispiel wird beim Betätigen der Stop-Taste immer dieselbe Kombination aus haptischer Rückmeldung und akustischer Rückmeldung ausgegeben. Selbstverständlich kann zusätzlich auch eine optische Rückmeldung ausgegeben werden. Da diese Art der Rückmeldung in Erwiderung auf die Betätigung der Stop-Taste nicht veränderbar ist, kann der Nutzer auch bei Bedienung einer anderen medizinischen Vorrichtung die Betätigung der Stop-Taste ohne große Überlegung validieren. Demzufolge kann für die medizinischen Vorrichtungen eines Unternehmens oder aber auch für die medizinische Vorrichtung eines bestimmten Typs, z.B. für Infusionspumpen, eine Standard-Rückmeldung etabliert werden. Auf diese Weise kann eine Sicherheit bei der Bedienung von medizinischen Vorrichtungen weiter erhöht werden.

In einer Weiterbildung kann die Rückmeldeeinheit so ausgebildet sein, dass sie auf eine Eingabebetätigung mit einem falschen Wert, einem unzulässigen Wert oder einer Fehleingabe eine, insbesondere jeweils, dedizierte Rückmeldung ausgibt. Dadurch wird der Anwender unmittelbar auf eine kritische Eingabebetätigung aufmerksam gemacht und eine Eingabe kann unmittelbar korrigiert werden.

Ferner können die Typen von Eingabebetätigungen in eine zweite Gruppe gruppiert sein. Die Rückmeldeeinheit kann dann so ausgebildet sein, dass eine Rückmeldung, die in Erwiderung auf einen Typ von Eingabebetätigung der zweiten Gruppe ausgegeben wird, festlegbar ist. In die zweite Gruppe können Typen von Eingabebetätigungen gruppiert werden, die sehr spezifisch sind und/oder auf die Zuverlässigkeit bzw. Sicherheit einer mit der medizinischen Vorrichtung durchgeführten Behandlung nur einen geringen oder keinen Einfluss haben. Beispiele hierfür sind eine Auswahlbetätigung zum Auswählen eines bestimmten Menüpunkts oder eine Umschaltung zwischen unterschiedlichen Anzeigen. Hierbei kann die Rückmeldung ganz individuell nur eine haptische oder akustische Rückmeldung sein. Auch kann die Kombination aus haptischer und akustischer Rückmeldung individuell festgelegt werden. Wiederum ist auch eine optische Rückmeldung oder eine Kombination mit einer optischen Rückmeldung denkbar. Selbstverständlich kann eine Rückmeldung für die zweite Gruppe auch ganz ausgeschaltet werden. Demnach kann ein Nutzer die Rückmeldungen, die in Erwiderung auf die Typen von Eingabebetätigungen der zweiten Gruppe ausgeben werden, ganz nach seinen individuellen Präferenzen festlegen. Durch diese individuelle Festlegung wird eine Vertrautheit beim Bedienen der medizinischen Vorrichtung und somit eine Sicherheit erhöht.

Die Eingabeeinheit kann bevorzugt einen Touchscreen aufweisen. Demnach kann ein häufiges Umschalten zwischen Anzeigen vermieden werden und eine Bedienung der medizinischen Vorrichtung wird vereinfacht. Bei ausschließlicher Verwendung des Touchscreens als Eingabeeinheit kann zudem ein übersichtliches Erscheinungsbild der medizinischen Vorrichtung erreicht werden. Zudem wird eine Reinigung oder Desinfektion einer medizinischen Vorrichtung, die einen Touchscreen aufweist, im Vergleich zu einer medizinischen Vorrichtung, die eine Mehrzahl von mechanischen Tasten aufweist, die Kanten und Rillen an der Außenfläche der medizinischen Vorrichtung bilden, vereinfacht bzw. verbessert. Aufgrund der Ausgabe einer Rückmeldung in Erwiderung auf eine Eingabebetätigung bietet der Touchscreen auch den Vorteil einer Taste, dass bei einer Eingabebetätigung eine haptische Wahrnehmung erfolgt. Dies wird in ähnlicher Weise auch durch eine akustische Rückmeldung erreicht. Der Touchscreen bietet zudem den weiteren Vorteil, dass er auch eine optische Rückmeldung ausgeben kann.

Mit Vorteil kann der Touchscreen eine ESD-Glasfront und/oder ESG-Glasfront oder eine chemische gehärtete Glasfront aufweisen. Diese Glasfronten bieten Vorteile in den Bereichen Robustheit, Biokompatibilität und Reinigung/Reinigungsmittel. Demnach kann eine zuverlässige und langlebige medizinische Vorrichtung erhalten werden.

Die Eingabeeinheit kann mindestens eine Taste aufweisen. Die Taste kann bevorzugt eine kapazitive Taste sein. Die Taste wird insbesondere für eine Eingabebetätigung verwendet, die durch einen Nutzer ohne langes Suchen in Menüs schnell auszuführen ist. Hierzu zählen beispielsweise das An/Aus-Schalten der medizinischen Vorrichtung oder das Stoppen einer mit der medizinischen Vorrichtung durchgeführten Behandlung. Durch das Vorsehen der Taste kann eine Sicherheit bei der Verwendung der medizinischen Vorrichtung verbessert werden.

Bevorzugt kann die Rückmeldeeinheit ein Vibrationselement aufweisen. Das Vibrationselement kann insbesondere ein Linear Resonant Actuator (LRA) sein. Auf diese Weise kann eine schnelle und intensive Rückmeldung in Erwiderung auf die Eingabebetätigung gegeben werden. Alternativ kann das Vibrationselement ein Vibrationsmotor oder ein Piezo-Aktuator sein. Piezo-Aktuatoren benötigen einen geringen Bauraum und stellen eine kurze Reaktionszeit bereit.

Besonders bevorzugt kann das Vibrationselement durch einen integrierten Schaltkreis implementiert sein. Auf diese Weise kann eine einfache Integration des Vibrationselements in die medizinische Vorrichtung erfolgen.

Darüber hinaus kann der integrierte Schaltkreis über einen Bus, insbesondere I²C (Inter-Integrated Circuit)-Bus, SPI (Serial Peripheral Interface)-Bus, One Wire-Bus, UART (Universal Asynchronous Receiver Transmitter)-Bus, CAN (Controller Area Network)-Bus, Bit-Bang (z.B. GPIO (General Purpose Input/Output)-Bus), ansteuerbar sein. Demnach ist die Integration des Vibrationselements in die medizinische Vorrichtung auf einfache Weise durchführbar.

Darüber hinaus kann die medizinische Vorrichtung bereits einen Lautsprecher und/oder eine Lichtquelle, insbesondere eine LED, und/oder einen Touchscreen aufweisen. Die Rückmeldeeinheit kann in diesem Fall durch Software, die ausgebildet ist, die Eingabebetätigung abzugreifen und in Erwiderung auf die abgegriffene Eingabebetätigung ein Signal zum Ausgeben der akustischen Rückmeldung an den Lautsprecher und/oder ein Signal zum Ausgeben der optischen Rückmeldung an die Lichtquelle und/oder an den Touchscreen auszugeben, realisiert sein. Demnach kann die Rückmeldeeinheit in einer bestehenden medizinischen Vorrichtung, die bereits einen Lautsprecher und/oder eine Lichtquelle und/oder einen Touchscreen aufweist, auf einfache Weise z.B. durch ein Firmware-Update nachgerüstet werden.

### Kurzbeschreibung der Figuren

Nachfolgend wird die medizinische Vorrichtung gemäß der vorliegenden Offenbarung unter Bezugnahme auf die beigefügten Figuren, in denen gleiche Elemente mit den gleichen Bezugszeichen versehen sind, beschrieben. Es zeigen:
Fig. 1 eine schematische Ansicht einer medizinischen Vorrichtung, die eine Eingabebetätigung von einem Nutzer empfängt und eine Rückmeldung in Erwiderung auf die Eingabebetätigung ausgibt;
Fig. 2 ein schematisches Blockschaltbild der medizinischen Vorrichtung; und
Fig. 3 ein Sequenzdiagramm für eine medizinische Vorrichtung, bei der die Rückmeldeeinheit zum Ausgeben einer akustischen Rückmeldung durch Software implementiert ist.

### Detaillierte Beschreibung der Figuren

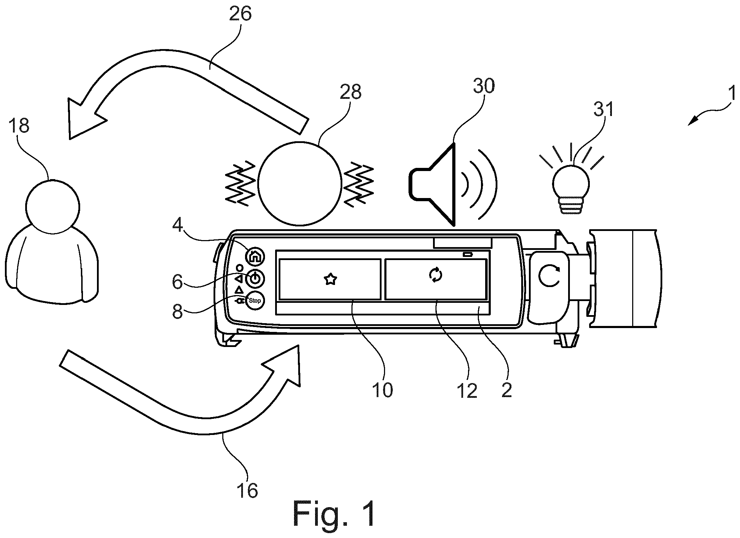

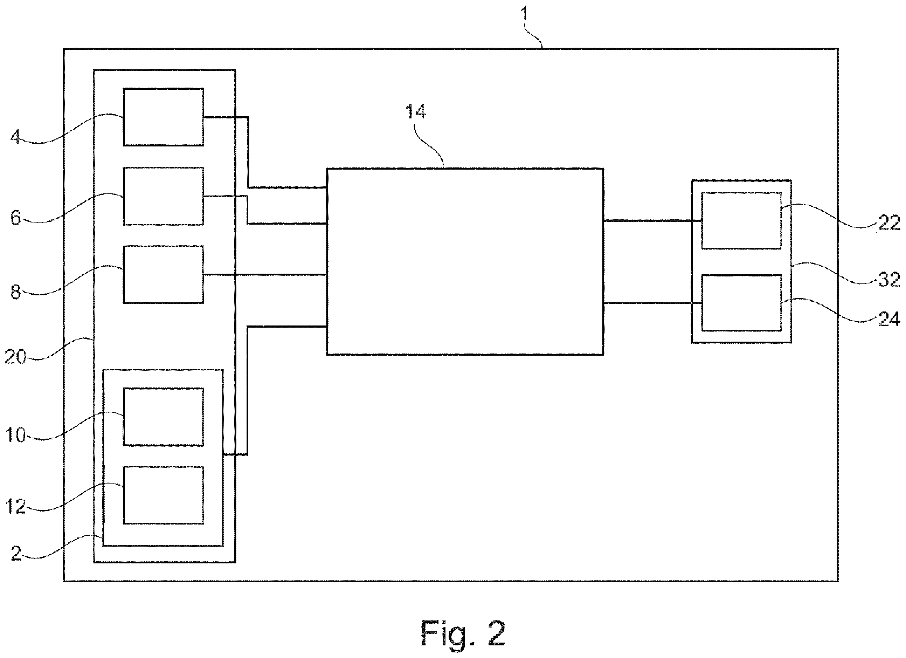

Fig. 1 zeigt eine offenbarungsgemäße medizinische Vorrichtung 1, die in der vorliegenden Ausführungsform als Infusionspumpe ausgebildet ist. Fig. 2 zeigt außerdem ein schematisches Blockschaltbild der medizinischen Vorrichtung 1. Die medizinische Vorrichtung 1 umfasst einen Touchscreen 2 und Tasten 4, 6, 8.

Der Touchscreen 2 ist so ausgebildet, dass er eine Berührung durch einen Finger oder durch ein anderes Eingabegerät, wie etwa einen Stift, empfängt, um eine entsprechende Verarbeitung in der medizinischen Vorrichtung 1 auszulösen. Auf dem Touchscreen 2 sind ein erstes Eingabesymbol 10 und ein zweites Eingabesymbol 12 angezeigt. Die Eingabesymbole 10, 12 werden durch Ausführen einer Software durch einen Mikrocomputer 14 der medizinischen Vorrichtung 1 auf dem Touchscreen 2 angezeigt und bei Berührung des Bereichs der Eingabesymbole 10, 12 auf dem Touchscreen 2 wird eine entsprechende Verarbeitung durch den Mikrocomputer 14 ausgeführt. In der vorliegenden Ausführungsform dient das erste Eingabesymbol 10 zum Auswählen oder Erstellen eines Profils eines neuen Patienten und das zweite Eingabesymbol 12 dient zum Auswählen des Profils desselben Patienten.

Die Tasten 4, 6, 8 sind bei der medizinischen Vorrichtung 1 aus Fig. 1 neben dem Touchscreen 2 und untereinander angeordnet. Vorliegend sind die Tasten 4, 6, 8 als eine Home-Taste 4, eine An/Aus-Taste 6 und eine Stop-Taste 8 ausgebildet. Die Tasten 4, 6, 8 bieten den Vorteil, dass sie ohne langes Suchen in einer auf dem Touchscreen 2 angezeigten Menüstruktur schnell betätigbar sind. Die Tasten 4, 6, 8 können hinter einer Folie oder einer Glasfläche angeordnet sein. Auf diese Weise werden Ränder oder Rillen, in denen sich Keime ansammeln können und die nicht zuverlässig zu reingingen oder desinfizieren sind, um die Tasten 4, 6, 8 herum vermieden.

Der Touchscreen 2 und die Tasten 4, 6, 8 sind ausgebildet, eine Eingabebetätigung 16, die durch einen Nutzer 18 der medizinischen Vorrichtung 1 ausgeführt wird, zu empfangen und an den Mikrocomputer 14 weiterzuleiten, so dass eine entsprechende Verarbeitung in dem Mikrocomputer 14 angestoßen wird. Infolge der Verarbeitung kann der Mikrocomputer 14 Signale ausgeben, um eine mit der medizinischen Vorrichtung 1 durchgeführte Behandlung zu steuern bzw. zu parametrieren. Der Touchscreen 2 und die Tasten 4, 6, 8 entsprechen somit gemäß der bevorzugten Ausführungsform zusammen, wie in Fig. 2 gezeigt, einer Eingabeeinheit 20. Es ist aber auch denkbar, dass die medizinische Vorrichtung 1 nur den Touchscreen 2 oder nur die Tasten 4, 6, 8 ggf. zusammen mit weiteren Tasten als die Eingabeeinheit 20 aufweist.

Die offenbarungsgemäße medizinische Vorrichtung 1 weist außerdem, wie in Fig. 2 gezeigt, ein Vibrationselement 22 und einen Lautsprecher 24 auf. Bei Empfang der Eingabebetätigung 14 über die Eingabeeinheit 20 wird gemäß der vorliegenden Offenbarung, wie in Fig. 1 gezeigt, eine Rückmeldung 26 an den Nutzer 18 ausgegeben. Wie in Fig. 2 gezeigt, wird die Ausgabe der Rückmeldung 26 ebenfalls durch den Mikrocomputer 14 bei Empfang der Eingabebetätigung 14 angestoßen. Die Rückmeldung 26 kann dabei eine haptische Rückmeldung 28 durch das Vibrationselement 22 oder eine akustische Rückmeldung 30 durch den Lautsprecher 24 sein. Darüber hinaus kann die Rückmeldung 26 auch eine Kombination aus akustischer und haptischer Rückmeldung 28, 30 sein. Das Vibrationselement 22 und der Lautsprecher 24 entsprechen somit zusammen, wie in Fig. 2 gezeigt, einer Rückmeldeeinheit 32. Es ist auch denkbar, dass die Rückmeldeeinheit 32 nur durch das Vibrationselement 22 oder nur durch den Lautsprecher 24 gebildet ist. Weiterhin ist denkbar, dass die Rückmeldung 26 zusätzlich oder alternativ zu der akustischen und/oder haptischen Rückmeldung 28, 30 eine optische Rückmeldung 31, beispielsweise in Form eines auf dem Touchscreen 2 angezeigten Lichtsignals oder einer Anzeige auf dem Touchscreen 2, ist. In diesem Fall wird die Rückmeldeeinheit 32 auch durch den Touchscreen 2 gebildet. Alternativ kann eine zusätzliche Lichtquelle, wie etwa eine LED, zum Ausgeben der optischen Rückmeldung 31 bereitgestellt sein. Die Lichtquelle ist dann ebenfalls Bestandteil der Rückmeldeeinheit 32.

Das Vibrationselement 22 ist vorliegend als Linear Resonant Actuator (LRA) ausgebildet. Alternativ kann das Vibrationselement 22 auch durch einen Vibrationsmotor oder einen Piezo-Aktuator gebildet sein. Es hat sich als bevorzugt erwiesen, wenn das Vibrationselement 22 durch einen integrierten Schaltkreis implementiert ist, der durch den Mikrocomputer 14 über einen Bus, wie etwa insbesondere einen I²C (Inter-Integrated Circuit)-Bus, einen SPI (Serial Peripheral Interface)-Bus, einen One Wire-Bus, einen UART (Universal Asynchronous Receiver Transmitter)-Bus, einen CAN (Controller Area Network)-Bus, Bit-Bang (z.B. einen GPIO (General Purpose Input/Output)-Bus), ansteuerbar ist.

Wie bereits erwähnt, weist die medizinische Vorrichtung 1 den Lautsprecher 24 auf. Der Lautsprecher 24 kann ebenfalls auf einem integrierten Schaltkreis bereitgestellt sein. Vorteilhafterweise kann der Lausprecher 24 auf demselben integrierten Schaltkreis wie das Vibrationselement 22 angeordnet sein.

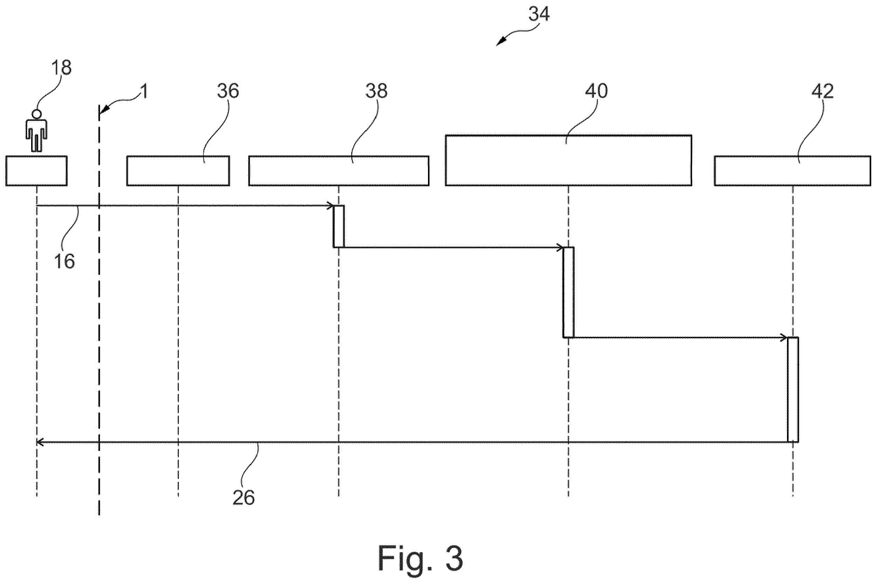

Viele medizinische Vorrichtungen 1 weisen jedoch bereits einen Lautsprecher 24 zum Ausgeben von Warnsignalen auf, so dass vorteilhaft ist, die Rückmeldeeinheit 32 zum Ausgeben der akustischen Rückmeldung 30 durch Software 34 zu realisieren. Ein Sequenzdiagramm der Software 34 ist in Fig. 3 gezeigt.

Wie in Fig. 3 gezeigt, tätigt ein Nutzer 18 eine Eingabebetätigung 16 auf dem Touchscreen 2, auf dem Eingabesymbole 10, 12 mittels einer Grafik-Schicht 36 angezeigt sind. Ein nachgeschalteter Machine User Interface Controller (MUIC) 38 erfasst dann eine Berührung des Touchscreens 2 im Bereich der Eingabesymbole 10, 12. Ein Event-Handler 40 greift die Erfassung der Eingabebetätigung 16 durch den MUIC 38 ab und veranlasst eine Audio-Steuerung 42, eine akustische Rückmeldung 30 über den Lautsprecher 24 auszugeben. Demnach kann die Rückmeldeeinheit 32 zum Ausgeben einer akustischen Rückmeldung 30 auf einfache Weise, z. B. durch ein Firmware-Update des Mikrocomputers 14, in einer medizinischen Vorrichtung 1, die bereits einen Lausprecher 24 aufweist, nachträglich implementiert werden. In ähnlicher Weise kann die medizinische Vorrichtung 1 bereits eine Lichtquelle, z.B. eine LED, oder den Touchscreen 2 aufweisen und es kann eine Beleuchtungs-Steuerung zusätzlich oder alternativ zu der Audio-Steuerung 42 implementiert werden, so dass eine optische Rückmeldung 31 ermöglicht wird.

In die Eingabeeinheit 20 werden, wie oben beschrieben, unterschiedliche Typen von Eingabebetätigungen 16 eingegeben. Die Rückmeldeeinheit 32 ist dabei so ausgebildet, das sie jeweils unterschiedliche Typen von Rückmeldungen 26 in Erwiderung auf die unterschiedlichen Typen von Eingabebetätigungen 16 ausgibt. In dem beschriebenen Beispiel kann eine Behandlung durch Drücken der Stop-Taste 8 unterbrochen werden. Dies stellt einen starken Eingriff in die mit der medizinischen Vorrichtung 1 durchgeführten Behandlung dar. Dahingegen stellt die Betätigung des Eingabesymbols 10 zum Anlegen eines Profils für einen neuen Patienten eine Verarbeitung dar, die vor der Durchführung der Behandlung erfolgt und somit kaum Einfluss auf die Behandlung hat. Folglich sollte vorzugsweise für die durch die Stop-Taste 8 getätigte Eingabebetätigung 16 ein anderer Typ von Rückmeldung 26 ausgegeben werden als für die durch das Eingabesymbol 10 getätigte Eingabebetätigung 16. Bei Betätigung der Stop-Taste 8 kann beispielsweise eine Kombination aus haptischer und akustischer Rückmeldung 28, 30 erfolgen, so dass die Rückmeldung durch zwei unterschiedliche Sinnesorgane erfassbar ist. Bei der zusätzlichen Ausgabe einer optischen Rückmeldung 31 z.B. über den Touchscreen ist eine Rückmeldung sogar durch drei Sinnenorgane erfassbar. Bei Betätigung des Eingabesymbols 10 hingegen kann zum Beispiel die Rückgabe einer haptischen Rückmeldung 28 ausreichen.

Darüber hinaus kann die Rückmeldeeinheit 28 ausgebildet sein, dass eine Intensität oder Frequenz der haptischen Rückmeldung 28 durch den Nutzer 18 festgelegt werden kann. In ähnlicher Weise kann eine Lautstärke der akustischen Rückmeldung 30 durch den Nutzer 18 festgelegt werden. Auf diese Weise kann der Nutzer die Rückmeldung 26 entsprechend seinen Präferenzen oder gemäß den Umgebungsbedingungen anpassen. In einer lauten Umgebung kann die Lautstärke erhöht werden, so dass die akustische Rückmeldung 30 hörbar ist. In ruhigen Umgebungen, wie etwa einem Operationssaal, kann die Lautstärke der akustischen Rückmeldung 30 reduziert werden und im Gegenzug kann die Intensität oder Frequenz der haptischen Rückmeldung 28 erhöht werden, so dass die Rückmeldung 26 zuverlässig erfassbar ist. Zusätzlich kann in einer stillen Umgebung die Ausgabe einer optischen Rückmeldung 31 vorteilhaft sein.

Zusätzlich können die Typen von Eingabebetätigungen 26 in eine erste Gruppe gruppiert sein. In die erste Gruppe können bevorzugt Typen von Eingabebetätigungen 16 gruppiert werden, die bei einer großen Anzahl von unterschiedlichen medizinischen Vorrichtungen 1 gleichsam vorhanden sind. Außerdem können in die erste Gruppe bevorzugt Typen von Eingabetätigungen 16 gruppiert werden, die eine große Relevanz für die Zuverlässigkeit bzw. die Sicherheit bei einer mit der medizinischen Vorrichtung 1 durchgeführten Behandlung haben. Die Rückmeldeeinheit 32 kann dann so ausgebildet sein, dass sie eine vorgegebene und nicht änderbare Rückmeldung 26 in Erwiderung auf einen Typ von Eingabebetätigung 16 der ersten Gruppe ausgibt. Beispielsweise kann für die Stop-Taste 8 eine vorgegebene Kombination von haptischer und akustischer Rückmeldung 28, 30 festgelegt sein. Diese Kombination kann durch einen Nutzer 18 nicht geändert werden. Demnach wird bei unterschiedlichen medizinischen Vorrichtungen 1 immer diese Rückmeldung 26 beim Betätigen der Stop-Taste 8 ausgegeben, so dass der Nutzer 18 mit der Rückmeldung 26 vertraut ist und eine Betätigung der Stop-Taste 8 zuverlässig validieren kann.

Die Rückmeldeeinheit 20 kann so ausgebildet sein, dass sie auf eine Eingabebetätigung 16 mit einem falschen Wert, einem unzulässigen Wert oder einer Fehleingabe eine dedizierte Rückmeldung 26 ausgibt. Dadurch wird der Anwender unmittelbar auf eine kritische Eingabebetätigung 16 aufmerksam gemacht und eine Eingabe kann unmittelbar korrigiert werden. Beispielsweise kann die Einstellung einer zu hohen oder zu niedrigen Dosierung zuverlässig und unmittelbar erkannt werden.

Die Typen von Eingabebetätigungen 16 können ferner in eine zweite Gruppe gruppiert sein. In die zweite Gruppe können Typen von Eingabebetätigungen 16 gruppiert werden, die sehr spezifisch sind und auf die Zuverlässigkeit bzw. Sicherheit einer mit der medizinischen Vorrichtung 1 durchgeführten Behandlung nur einen geringen oder keinen Einfluss haben. Die Rückmeldeeinheit 28 kann dann so ausgebildet sein, dass eine Rückmeldung 26, die in Erwiderung auf einen Typ von Eingabebetätigung 16 der zweiten Gruppe ausgegeben wird, festgelegt werden kann. Im vorliegenden Beispiel können Eingabebetätigungen 16, die über die Eingabesymbole 10 und 12 getätigt werden, in die zweite Gruppe gruppiert sein, da deren Betätigung vor einer eigentlichen Behandlung des Patienten erfolgt. Der Nutzer 18 kann die Rückmeldung 26, die in Erwiderung auf die Betätigung der Eingabesymbole 10, 12 ausgegeben wird, entsprechend seinen Präferenzen anpassen, so dass eine Vertrautheit mit der medizinischen Vorrichtung 1 erhöht wird. Beispielsweise kann die Rückmeldung 26 als eine haptische Rückmeldung 28 festgelegt werden, um störende Geräusche zu vermeiden. Alternativ kann die Ausgabe einer Rückmeldung 26 auch ganz ausgeschaltet werden.

Die medizinische Vorrichtung 1 ermöglicht es somit, eine Sicherheit beim Bedienen der medizinischen Vorrichtung 1 durch Ausgeben der Rückmeldung 26 in Erwiderung auf die Eingabebetätigung 16 zu erhöhen. Durch die Ausgabe von Standard-Rückmeldungen in Erwiderung auf bestimmte Eingabebetätigungen, die in medizinischen Vorrichtungen 1 oftmals vorhanden sind und/oder einen großen Einfluss auf eine mit der medizinischen Vorrichtung 1 durchgeführte Behandlung haben, kann der Nutzer 18 die gemachte Eingabebetätigung 16 sicher erfassen bzw. validieren. Außerdem wird durch die Einstellbarkeit der Rückmeldung 26, die in Erwiderung auf eine weniger relevante Eingabebetätigung 16 ausgegeben wird, eine Vertrautheit des Nutzers 18 mit der medizinischen Vorrichtung 1 gesteigert, wodurch eine Sicherheit ebenfalls erhöht wird.

Vorliegend wurde die medizinische Vorrichtung 1 als eine Infusionspumpe beschrieben. Die vorliegende Offenbarung ist nicht darauf begrenzt und kann auch für andere medizinische Vorrichtungen 1, bei denen Eingabebetätigungen durchführbar sind, angewendet werden.

### Bezugszeichenliste

- 1: medizinische Vorrichtung
- 2: Touchscreen
- 4: Home-Taste
- 6: An/Aus-Taste
- 8: Stop-Taste
- 10: erstes Eingabesymbol
- 12: zweites Eingabesymbol
- 14: Mikrocomputer
- 16: Eingabebetätigung
- 18: Nutzer
- 20: Eingabeeinheit
- 22: Vibrationselement
- 24: Lautsprecher
- 26: Rückmeldung
- 28: haptische Rückmeldung
- 30: akustischer Rückmeldung
- 31: optische Rückmeldung
- 32: Rückmeldeeinheit
- 34: Software
- 36: Grafik-Schicht
- 38: Machine User Interface Controller (MUIC)
- 40: Event-Handler
- 42: Audio-Steuerung

## Patentansprüche

1. Medizinische Vorrichtung (1), die aufweist:
eine Eingabeeinheit (20), die ausgebildet ist, eine durch einen Nutzer (18) getätigte Eingabebetätigung (16) zum Bedienen der medizinischen Vorrichtung (1) zu empfangen;
**gekennzeichnet durch**
eine Rückmeldeeinheit (32), die ausgebildet ist, dem Nutzer (18) in Erwiderung auf die Eingabebetätigung (16) eine haptische Rückmeldung (28) und/oder eine akustische Rückmeldung (30) und/oder eine optische Rückmeldung (31) auszugeben.

2. Medizinische Vorrichtung (1) gemäß Anspruch 1, **dadurch gekennzeichnet, dass**
die Eingabeeinheit (20) ausgebildet ist, unterschiedliche Typen von Eingabebetätigungen (16) zu empfangen; und
die Rückmeldeeinheit (32) ausgebildet ist, jeweils unterschiedliche Typen von Rückmeldungen (26) in Erwiderung auf die unterschiedlichen Typen von Eingabebetätigungen (14) auszugeben.

3. Medizinische Vorrichtung (1) gemäß einem der Ansprüche 1 oder 2 **dadurch gekennzeichnet, dass**
die Rückmeldeeinheit (32) ausgebildet ist, dass eine Intensität oder Frequenz der haptischen Rückmeldung (28) und/oder eine Lautstärke der akustischen Rückmeldung (30) und/oder Helligkeit der optischen Rückmeldung (31) festlegbar ist.

4. Medizinische Vorrichtung (1) gemäß einem der Ansprüche 2 oder 3, **dadurch gekennzeichnet, dass**
die Typen von Eingabebetätigungen (14) in eine erste Gruppe gruppiert sind; und
die Rückmeldeeinheit (32) ausgebildet ist, eine vorgegebene und nicht änderbare Rückmeldung (26) in Erwiderung auf einen Typ von Eingabebetätigung (14) der ersten Gruppe auszugeben.

5. Medizinische Vorrichtung (1) gemäß einem der Ansprüche 2 bis 4, **dadurch gekennzeichnet, dass**
die Rückmeldeeinheit so ausgebildet ist, dass sie auf eine Eingabebetätigung mit einem falschen Wert, einem unzulässigen Wert oder einer Fehleingabe eine, insbesondere jeweils, dedizierte Rückmeldung ausgibt.

6. Medizinische Vorrichtung (1) gemäß einem der Ansprüche 2 bis 5, **dadurch gekennzeichnet, dass**
die Typen von Eingabebetätigungen (14) in eine zweite Gruppe gruppiert sind; und
die Rückmeldeeinheit (32) ausgebildet ist, dass eine Rückmeldung (26), die in Erwiderung auf einen Typ von Eingabebetätigung (14) der zweiten Gruppe ausgegeben wird, festlegbar ist.

7. Medizinische Vorrichtung (1) gemäß einem der Ansprüche 1 bis 6, **dadurch gekennzeichnet, dass**
die Eingabeeinheit (20) mindestens eine Taste (4, 6, 8), insbesondere eine kapazitive Taste, aufweist.

8. Medizinische Vorrichtung (1) gemäß einem der Ansprüche 1 bis 7, **dadurch gekennzeichnet, dass**
die Rückmeldeeinheit (32) ein Vibrationselement (24), insbesondere einen Linear Resonant Actuator (LRA) oder einen Piezo-Aktuator, aufweist.

9. Medizinische Vorrichtung (1) gemäß Anspruch 8, **dadurch gekennzeichnet, dass**
das Vibrationselement (24) durch einen integrierten Schaltkreis implementiert ist.

10. Medizinische Vorrichtung (1) gemäß Anspruch 9, **dadurch gekennzeichnet, dass**
der integrierte Schaltkreis über einen Bus, insbesondere I²C (Inter-Integrated Circuit)-Bus, SPI (Serial Peripheral Interface)-Bus, One Wire-BUS, UART (Universal Asynchronous Receiver Transmitter)-Bus, CAN (Controller Area Network)-Bus, Bit-Bang, ansteuerbar ist.

11. Medizinische Vorrichtung (1) gemäß einem der Ansprüche 1 bis 10, **dadurch gekennzeichnet, dass**
die medizinische Vorrichtung (1) einen Lautsprecher (24) und/oder eine Lichtquelle und/oder einen Touchscreen (2) aufweist, und
die Rückmeldeeinheit (32) durch Software (34), die ausgebildet ist, die Eingabebetätigung (14) abzugreifen und in Erwiderung auf die abgegriffene Eingabebetätigung (14) ein Signal zum Ausgeben der akustischen Rückmeldung (30) an den Lautsprecher (24) und/oder ein Signal zum Ausgeben der optischen Rückmeldung (31) an die Lichtquelle und/oder an den Touchscreen (2) auszugeben, realisiert ist.
